# EUROPEAN PATENT APPLICATION

(11) **EP 3 106 129 A1**
(43) Date of publication of application: **21.12.2016**
(21) Application number: 15172308.7
(22) Date of filing: 16.06.2015
(51) Int. Cl.: A61F 2/24

(54) **HEART VALVE STENT WITH VARIABLE THICKNESS**

(71) Applicant: Epygon Sasu, 75008 Paris (FR)
(72) Inventor: PASQUINO, Enrico, 10020 Marentino (IT); SCORSIN, Marcio, 10125 Torino (IT); GARD, Marco, 10031 Borgomasino (Torino) (IT); CASALEGNO, Sergio, 10099 San Mauro Torinese (Torino) (IT)
(74) Representative: Grosfillier, Philippe

(57) **Abstract**

Heart valve stent having a general tubular shape, characterized by the fact that it has a variable thickness.

## Description

### Field of invention

The present invention generally relates to a valvular stent. It more precisely relates to a stent for a sutureless or transcatheter heart valve prosthesis designed to be implanted at level of an aortic, pulmonary, mitral or tricuspid valve.

### State of the art

In the last decades, since the bioprosthesis have been developed, different types of heart valve stents have been designed, with different materials and geometries. The conventional sutured surgical bioprostheses adopt a stent in order to provide the necessary mechanical support to the biological functional component of the bioprosthesis. The stent, in fact, is granting the long-term fatigue resistance of the prosthesis minimizing the risk of tissue tears. The introduction in clinical use of the sutureless and transcatheter heart valves has expanded the function of the stent since these types of bioprostheses don't require to be sutured to the patient's heart. The stents, designed for sutureless and transcatheter heart valves, must also grant a safe and functional anchoring of the prosthesis to the surrounding heart tissues where the prosthesis is being implanted.

It turns out that the valvular stent, in such prostheses, has a complex mechanical function that must satisfy the need of an external anchoring and an adequate protection of the valve's bio-functional component.

This type of stent has a general tubular shape and requires a safe anchoring/fitting to the heart anatomy in order to prevent migration during the cardiac cycle and to minimize the occurrence of paravalvular leakages. Several other requirements have to be met. In particular the dynamic functionality has to be preserved.

The stent wall is in contact with different anatomical areas such as the atrium wall, annulus, ventricle or aortic wall. The interactions, in particular the forces, between the stent and those areas and may considerably vary along and around the stent wall.

If a part of the stent is in the atrium it must apply a certain pressure on the atrial floor in order to minimize the risk of leakage. But at the same time must be flexible enough to fit the anatomy without causing damages to the tissues.

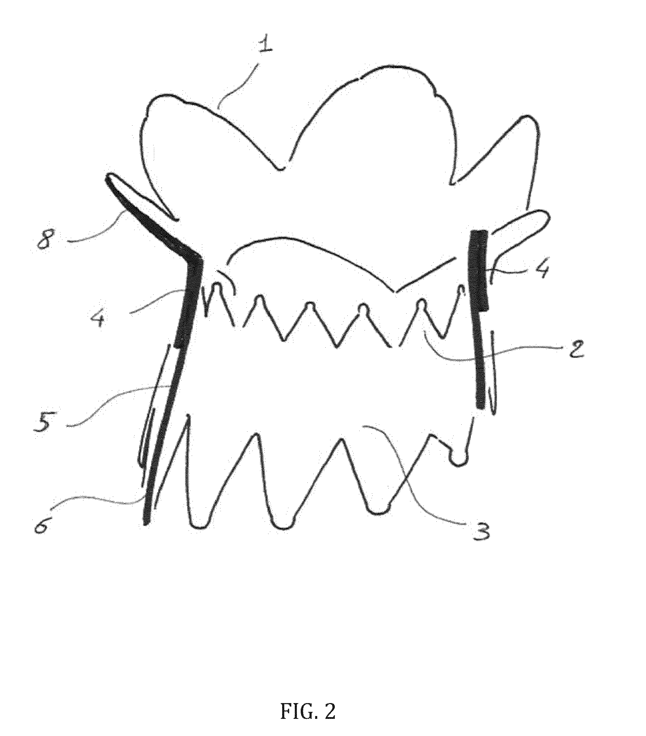

The interaction between the stent annular portion with the native mitral fibrous annulus is quite critical, in particular for the clinical outcomes of an atrio-ventricular valve (see figure 2).

The stent design in this annular portion must well fit the native annulus with a geometry that will respect the anatomy but in the same time will apply an outward force that can be modulated along the circumference and differentiated having an antero-posterior outward force different from the commissure-commissure force. In this case, since in atrio-ventricular valves, for example the mitral valve, the demand in outward force along the annulus circumference depends from the underlying anatomical structures. The so called "posterior portion" can receive a pressure, from the stent annular ring, higher than the "anterior portion" where the presence of the mitro-aortic continuity can influence and interfere with the fluidodynamic of the aortic valve and create septal compressions with rhythm conduction blocks. This case can be easily extended to other native valves such as tricuspid, aortic and pulmonary ones.

The design of the existing heart valve stents does not allow to sufficiently take into account the previous cited various interactions. In some locations the stent is either too rigid or too flexible.

There is therefore a need to improve the existing heart valve stents.

### Description of the invention

The problems mentioned in the previous chapter are solved or at least partially solved, in the present invention. The stent has a general tubular shape and is characterized by the fact that it has a variable thickness.
Figures 1A, 1B and 2 represents mitral valve stents according to the invention. The atrial side is in the upper part and the ventricular side in the lower part of the figures. A black line of varying thickness represents the wall thickness.
Figure 3 shows an example of a stent cross-section according to invention, with a wall of variable thickness around its circumference.
Figures 4a to 4h illustrate longitudinal sections of various stent designs according to the invention.
Figures 5a to 5f illustrate longitudinal sections of changes in thickness.

### Numerical references used in the figures

- 1.: Atrial region
- 2.: Annular region
- 3.: Ventricular region
- 4.: Stent annular portion
- 5.: Upper stent ventricular portion
- 6.: Lower stent ventricular portion
- 7.: Continuous thickness variation
- 8.: Stent atrial flanges
- 9.: Stent posterior wall
- 10.: Stent anterior wall

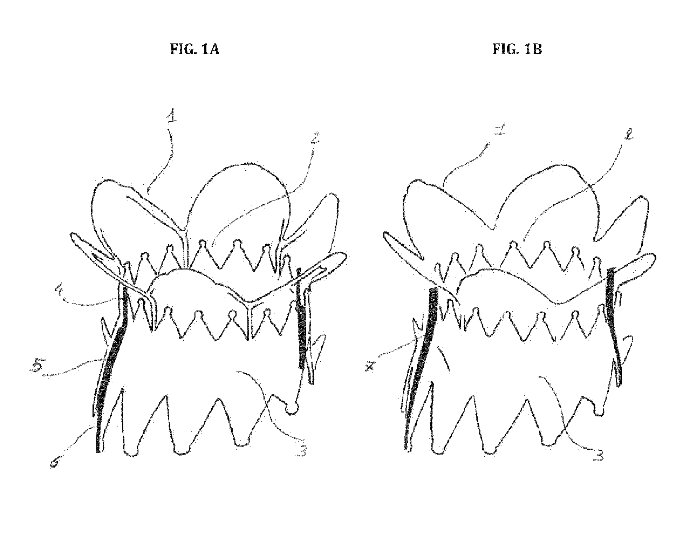

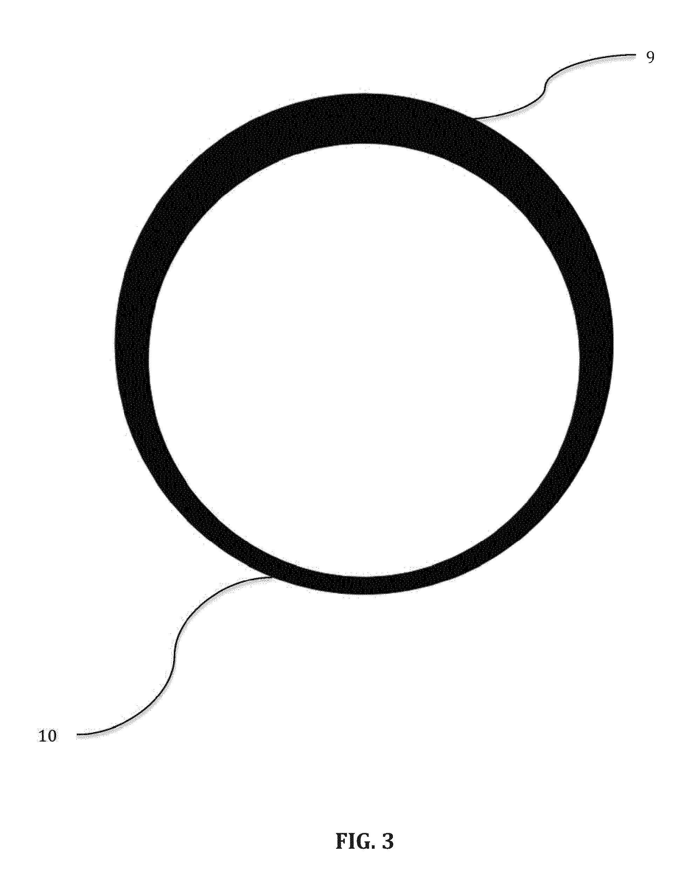

The thickness may vary radially, i.e. along its circumference (see figure 3), and/or longitudinally, i.e. along its length (figures 1A, 1B and 2).

In the stent illustrated on figure 1A, the wall is relatively thick on the upper ventricular side **5** and thinner at the annulus level **4** and at the lower ventricular side **6.**

In a general manner, the stent wall is thicker in a stent location where the resistance and/or rigidity with respect to the surrounding tissues needs to be important and thinner in the locations where the stent needs to be more flexible.

If the stent has to be used with a mitral valve the posterior part may be thicker and consequently stiffer than the anterior part or vice versa depending on the stent design.

The atrial portion of the stent can have a variable design geometry including a variable thickness that can, for example, decrease from the annulus to the most peripheral portion of the stent's atrial flange **8.**

In the ventricular portion **3** of a valvular stent for atrio-ventricular valve the anatomical conditions are also asymmetric. The portion of the stent coming in contact with the cardiac muscle must have a design and a thickness suitable to absorb the mechanical forces provided by the myocardial muscle, to protect the biomechanics of the prosthetic valve and to grant a long-term resistance of the material to fatigue. These last requirements are quite complex from the engineering point of view and can be addressed not only varying the width of the stent structure but more advantageously in varying the stent thickness. For example in the case of a mitral valve the stent may be provided with a decreasing thickness from annular ring **4** distally towards ventricle apex **5, 6** and/or radially antero-posterior **9,10.**

A varying thickness may also be applied to the design of extension bodies that are created in the stent structure and which act as anchoring means to reduce the risk of valve migration and to eliminate possible interferences of the sub-valvular region.

A common process to make a heart valve stent is to cut out the design from a tube typically made of a metal alloys or a plastic polymer.

The same process can be used in the present invention and more generally any suitable process can be used to manufacture a stent according to the present invention.

The variable thickness of the stent can be obtained starting from a tube with a longitudinal or circumferential variable thickness. The invention requires that the variable thickness is located on the tube at specific coordinates where later on the stent structures will be cut.

Three non-limiting processes are briefly presented below.

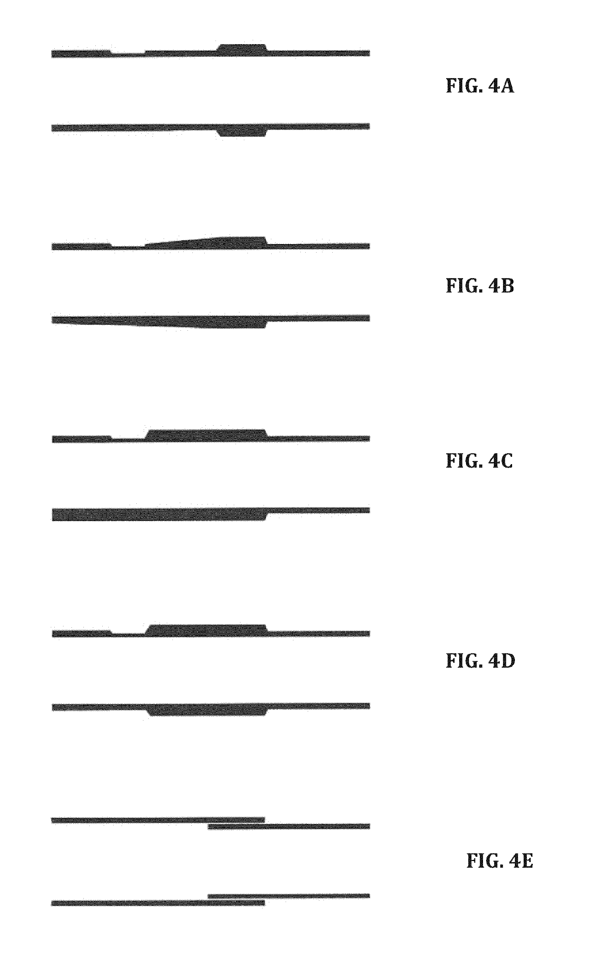

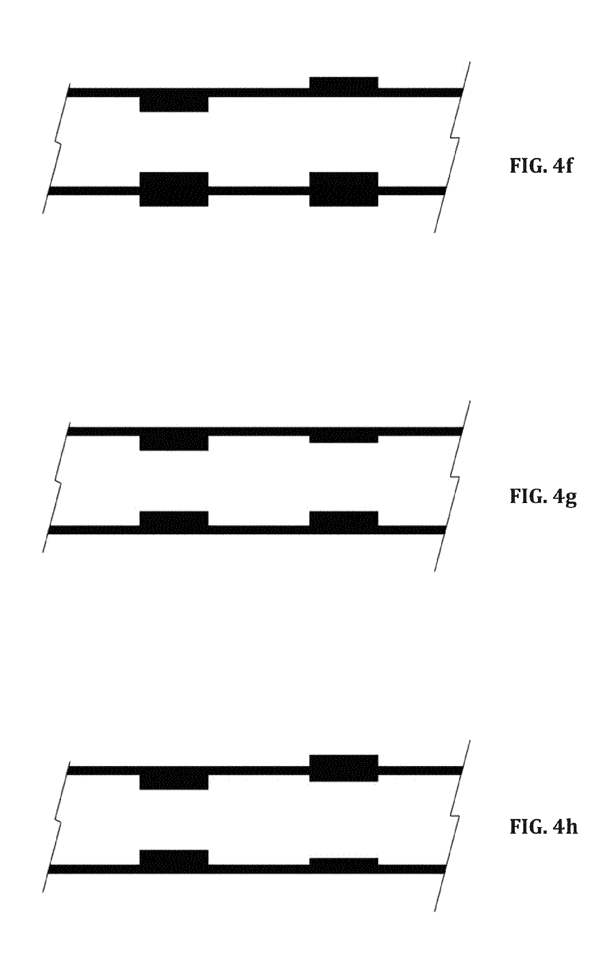

The first one is to start with a tube with a thickness that is equal to the thicker structure that must be obtained. With mechanical or laser tooling techniques the tube wall thickness is reduced from outside or from inside. Figures 4a to 4d are cross sections of different tubes obtained according show some examples of this first process.

In an alternative approach the wall thickness is reduced before the tube formation, when the wall is still flat.

The second process comprises a step in which one or more tube rings or geometrically shaped portions of a ring are provided inside or outside of the tube, those additional elements are then fixed to the tube. Any suitable technique can be used for the fixation such as gluing, electric or laser welding, sinterization process. The stent illustrated on figures 4e is obtained according to this second process.

The third process is based on drawing and forging metal tubes with a variable thickness.

In the second and the third process a further adjustment of the variable thickness areas can be obtained with mechanical tooling or laser procedures.

It is also possible to combine the 3 processes and thereby obtain complex design geometries such as the one illustrated on figures 4f to 4h.

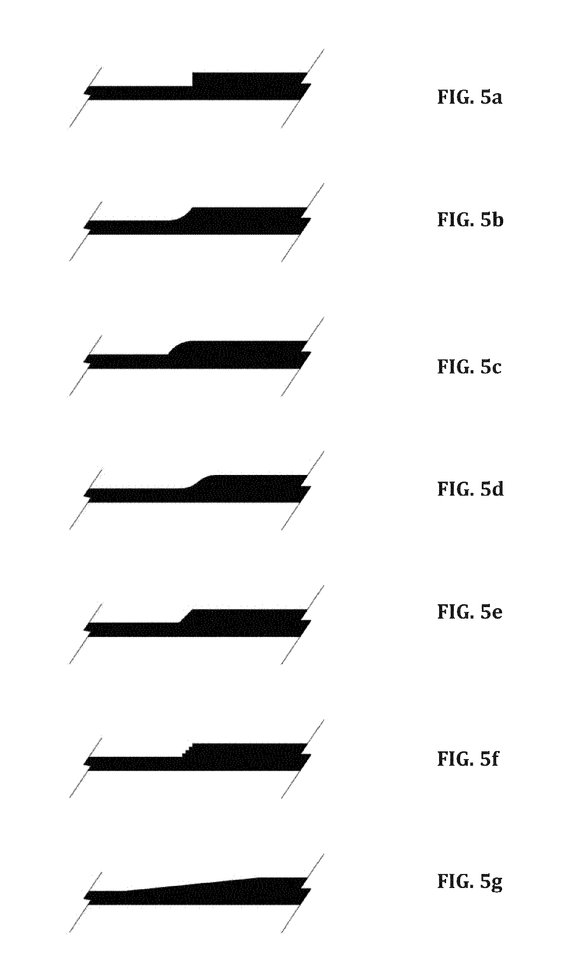

The thickness variation can be continuous (e.g. linear or parabolic) or discontinuous. Figures 5a to 5g show some examples of such variations.

In general a continuous thickness variation is preferred if the change in the stent resistance has to be progressive. On the other end the thickness variation will be discontinuous if an important change of stent resistance is needed between two close locations.

## Claims

1. Heart valve stent having a general tubular shape, **characterized by** the fact that it has a variable thickness.

2. Heart valve stent according to claim 1 wherein the thickness varies radially.

3. Heart valve stent according to claim 1 or 2 wherein the thickness varies longitudinally.

4. Heart valve stent comprising an annulus portion which is approximately located in the middle of the stent and which therefore separate the tube in two portions, e.g. an atrial and a ventricular portions, wherein the thickness of at last one portion decreases from the annulus side to its opposite side.

5. Mitral valve stent according the previous claim comprising an annulus portion and an atrial portion, wherein the thickness of the atrial portion decreases from its annulus side to its opposite side.

6. Mitral valve stent according to anyone of the previous claims comprising an annulus portion and a ventricular portion, wherein the thickness of the ventricular portion decreases from its annulus side to its opposite side.

7. Mitral valve stent according to anyone of the previous claims **characterized by** a posterior part that is thicker than the anterior part.

8. Heart valve stent according to anyone of the previous claims wherein the thickness varies continuously.

9. Heart valve stent according to anyone of claims 1 to 7 wherein the thickness varies discontinuously.

10. Process for manufacturing a heart valve stent as defined in anyone of the previous claims comprising a first step in which stent wall is provided with a uniform thickness that correspond to the largest thickness of the final product and a second step **characterized by** a thickness reduction in specific stent locations.

11. Process according to claim 10 wherein the thickness reduction is being made before the tube formation.
